Europäisches Patentamt

European Patent Office

Office européen des brevets

(11) Veröffentlichungsnummer : **0 150 198**
**B1**

(12) EUROPÄISCHE PATENTSCHRIFT

(45) Veröffentlichungstag der Patentschrift :
13.04.88

(51) Int. Cl.⁴ : **A 61 F   2/34**

(21) Anmeldenummer : **84902710.7**

(22) Anmeldetag : **11.07.84**

(86) Internationale Anmeldenummer :
**PCT/EP 84/00210**

(87) Internationale Veröffentlichungsnummer :
**WO/8500284 (31.01.85 Gazette 85/03)**

(54) HÜFTPFANNEN-ENDOPROTHESE.

(30) Priorität : **14.07.83 DE 3325448**

(43) Veröffentlichungstag der Anmeldung :
**07.08.85 Patentblatt 85/32**

(45) Bekanntmachung des Hinweises auf die Patenterteilung : **13.04.88 Patentblatt 88/15**

(84) Benannte Vertragsstaaten :
**AT BE CH DE FR GB LI LU NL SE**

(56) Entgegenhaltungen :
**EP-A- 0 091 315**
**FR-A- 2 387 641**
**GB-A- 2 052 997**
**US-A- 3 818 512**

(73) Patentinhaber : **Waldemar Link GmbH & Co**
**Barkhausenweg 10**
**D-2000 Hamburg 63 (DE)**

(72) Erfinder : **KELLER, Arnold**
**An der Naherfurth 5**
**D-2061 Kayhude (DE)**

(74) Vertreter : **Glawe, Delfs, Moll & Partner Patentanwälte**
**Postfach 26 01 62 Liebherrstrasse 20**
**D-8000 München 26 (DE)**

## Beschreibung

Die Erfindung bezieht sich auf eine Hüftpfannen-Endoprothese.

Bei einer bekannten Endoprothese (DE-A 29 50 536) besteht die Fassung aus einer Metallschale, die auf der Außenseite Gewindegänge zur Verankerung im Acetabulum aufweist und deren zur Aufnahme des Einsatzes bestimmte Höhlung von einem Konus und einer im wesentlichen ebenen Bodenfläche begrenzt ist. Entsprechend wird die Außengestalt des Einsatzes durch eine Konusfläche und eine im wesentlichen ebene Abschlußfläche gebildet, wobei diese Flächen bei Fassung und Einsatz als Paßflächen übereinstimmend ausgebildet sind. Am Rand weisen Fassung und Einsatz gleichmäßig über den Umfang verteilte Vorsprünge und Vertiefungen zur Sicherung der jeweils gewählten Winkeleinstellung auf. Der Einsatz enthält eine die Gelenkfläche bildende, etwa halbsphärische Höhlung, deren Öffnungsbegrenzung durch die Kante zur Stirnfläche des Einsatzes gebildet wird. Die Öffnungsbegrenzungen der Gelenkfläche und der Fassung liegen in parallelen Ebenen. Die Mittelachse der Gelenkfläche verläuft exzentrisch parallel zur Mittelachse der Paßflächen, die der Einfachheit halber als Fassungsmittelachse bezeichnet wird. Durch unterschiedliche Winkeleinstellung des Einsatzes in der Fassung läßt sich die Lage der Gelenkfläche in bezug auf die eingesetzte Fassung verändern, um beispielsweise Ungenauigkeiten, die beim Einsetzen der Fassung vorgekommen sein mögen, auszugleichen. — Die praktische Bedeutung dieser Einstellmöglichkeit ist gering, weil die Einstellungsdifferenzen gering sind ; denn die Exzentrizität der Gelenkfläche kann nur gering sein, weil man die Fassung mit möglichst geringen Abmessungen ausstattet.

Ferner ist es bei prothetischen Hüftpfannen, die unmittelbar und ohne nachträgliche Verstellbarkeit im Acetabulum implantiert werden, bekannt (GB-A-2 052 997) den Rand der Hüftpfanne mit unterschiedlich gerichteten Schrägflächen zu versehen, um dadurch eine bessere Anpassung an die natürliche Form des knöchernen Acetabulums und einen größeren Spielraum für die femurseitigen Gelenkteile zu erreichen. Daraus ergibt sich ein von der ebenen Gestalt abweichender Verlauf des Öffnungsrands der Gelenkfläche, ohne daß dadurch die Mittelachsrichtung der Gelenkfläche variierend beeinflußt werden kann.

Die Erfindung hat erkannt, daß die Weiterbildung der oben an erster Stelle geannten, bekannten Endoprothese zu beträchtlichen Vorteilen führen kann, wenn es darum geht, die Richtung des Gelenks gegenüber der durch das Acetabulum vorgegebenen Gelenkrichtung oder optimalen Verankerungsrichtung zu korrigieren, um dadurch die Luxationsgefahr herabzusetzen. Demnach liegt für die Bestimmungsstaaten AT, BE, LU, NL und SE die Aufgabe der Erfindung in der Schaffung einer Endoprothese, die es erlaubt, die Winkelstellung der Fassung im knöchernen Acetabulum primär nach der optimalen Verankerung auszurichten, während die günstigste, luxationssichere Ausrichtung der Gelenkfläche gegenüber dem Prothesenkopf durch die Auswahl und die Ausrichtung des Einsatzes In der Fassung erreicht wird.

Diese Aufgabe wird durch die im Anspruch 1 gemäß Anspruchsfassung A angegebenen Merkmale gelöst.

Insbesondere bei dysplastischen Hüftverhältnissen kann man auf diese Weise oftmals noch eine sichere Verankerung der Fassung im Acetabulum erreichen, die nicht möglich wäre, wenn man die Fassung auf die beabsichtigte Gelenkrichtung im Acetabulum auszurichten hätte.

Bei einer bevorzugten Ausführungsform der Erfindung schließt die zur Öffnungsbegrenzung der Gelenkfläche lotrechte Mittelachse einen spitzen Winkel mit der Fassungsmittelachse ein.

Ein sicherer Sitz des Einsatzes in der Fassung und eine dennoch einfache Herstellbarkeit der Teile kann dann erreicht werden, wenn die zusammenwirkenden Paßflächen an der Fassung und am Einsatz von der sphärischen Form abweichende Rotationsflächen (beispielsweise Konusflächen) um die Fassungsmittelachse sind und wenn die Fassung und der Einsatz mit ineinandergreifenden Erhöhungen und Vertiefungen zur Sicherung der gewählten Winkeleinstellung versehen sind, wobei die Erhöhungen und Vertiefungen am äußeren Rand einer die Rotationsfläche abschließenden Basisfläche liegen, wobei die Basisfläche in der Tiefe der Fassung bzw. am hinteren Ende des Einsatzes angeordnet ist.

Für die Bestimmungsstaaten Bundesrepublik Deutschland, Schweiz, Liechtenstein, Frankreich und Großbritannien gehört eine Hüftpfanne nach der EP-A-0 091 315 zum Stand der Technik gemäß Art. 54 (1) (3) (4) EPÜ. Für diese Länder wird daher Schutz ausschließlich im Umfang des einzigen Anspruchs gemäß Anspruchsfassung B begehrt. Bei der bekannten Hüftpfanne setzen sich die zusammenwirkenden Paßflächen der Fassung und des Einsatzes aus polygonal zueinander angeordneten, parallel zur Fassungsmittelachse verlaufenden, randnah gelegenen Flächen sowie einer sich basisseitig daran anschließenden sphärischen Fläche zusammen. Es ist schwer, die polygonalen Flächen mit großer Genauigkeit herzustellen. Das bedeutet, daß das Zusammenwirken des Einsatzes und der Fassung gerade im randnahen Bereich, in welchem die größten Kräfte zu übertragen sind, mit beträchtlichen Maßtoleranzen behaftet ist, was zu unsicherem Sitz führen kann. Bei der bekannten Hüftpfanne gemäß DE-A-29 50 536 wird ein insgesamt besserer Sitz durch die Verwendung von Rotationsflächen erreicht, die von der sphärischen Form abweichen. Jedoch sind auch dort die Erhöhungen und Vertiefungen, die zur gegenseitigen Verdrehsicherung von Einsatz und Fassung vorgesehen sind, nahe dem Öffnungsrand angeordnet, wo sie sich ungünstig auf die Kraftübertragung auswirken. — Schließlich ist eine Hüftpfanne bekannt (US-A-3 818 512), deren Einsatz mit der Fassung über Rotationsflächen zusammenwirkt, die von der sphärischen Form abweichen und im randfernen Bereich angeordnet sind, während im randnahen Bereich eine Gewindemutter mit im Längsschnitt keilförmiger Fläche auf den Außenrand des Einsatzes einwirkt, der zwecks Nachgiebigkeit gegenüber den Schraubkräften geschlitzt ist, wodurch er geschwächt wird.

Der Erfindung liegt somit für die Bestimmungsstaaten DE, CH, LI und FR die Aufgabe zugrunde, eine Hüftpfanne zu schaffen, die bei einfacher Formgebung trotz variierbarer Winkeleinstellung des Einsatzes gegenüber der Fassung sicheren Sitz und zuverlässige Kraftübertragung gewährleistet. Die erfindungsgemäße Lösung findet sich im Gegenstand des einzigen Anspruchs gemäß Anspruchsfassung B.

Die Erfindung wird im folgenden näher unter Bezugnahme auf die Zeichnung erläutert, die ein vorteilhaftes Ausführungsbeispiel veranschaulicht. Es zeigen :

Fig. 1 einen Axialschnitt durch die Prothese in vergrößertem Maßstab,

Fig. 2 und 3 einen Axialschnitt und eine Draufsicht der Prothesenfassung und

Fig. 4 und 5 einen Axialschnitt und eine Draufsicht des Protheseneinsatzes.

Die Prothese besteht aus zwei Teilen, nämlich der Fassung 1 und dem Einsatz 2. Die Fassung besteht aus festem Material wie Metall oder Keramik und weist an ihrer Außenfläche Vorsprünge 3 auf, die zur Verankerung im Knochengewebe bestimmt sind und vorzugsweise als selbstschneidendes Gewinde ausgeführt sind. Ihre hintere, äußere Basisfläche 4 ist im wesentlichen eben.

Die Innenfläche der Fassung 1 wird an den Seiten überwiegend von einer Konusfläche 5 sowie nahe dem Öffnungsrand von einer zylindrischen Fläche 6 gebildet. Die innere Basisfläche 7 ist im wesentlichen eben und durch eine zentrische Bohrung mit Rand 19 auf eine Kreisringfläche reduziert. Diese Flächen sind Rotationsflächen in bezug auf die Fassungsmittelachse 9. Die Basisfläche 7 trägt an einer Stelle einen zylindrischen, zur Fassungsmittelachse 9 parallelen Zylindervorsprung 10. Die Stirnfläche 11 am Öffnungsrand der Fassung liegt in einer Ebene, die lotrecht zur Symmetrieachse 9 steht. Das bedeutet nicht, daß die Stirnfläche stets in einer Ebene liegen muß ; vielmehr kann Sie auch gekrümmt oder aus mehreren im Winkel aneinandergrenzenden Flächen zusammengesetzt sein. Sie ist aber vorzugsweise eine Rotationsfläche zur Fassungsmittelachse 9.

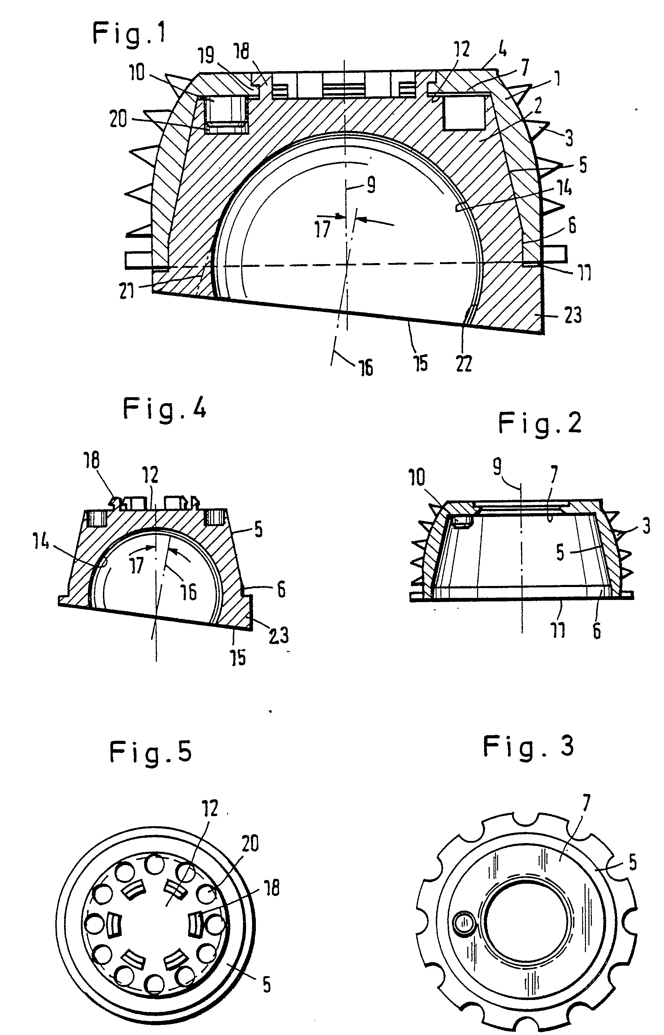

Der Einsatz 2 besteht aus einem im Hinblick auf die Gelenkfunktion reibungsarmen Werkstoff wie Kunststoff, beispielsweise Polyäthylen hoher Dichte. Seine seitliche Außenfläche ist durch dieselbe Konus- bzw. Zylinderfläche 5, 6 bestimmt wie die Innenfläche der Fassung. Die Basisfläche 12 ist eben wie diejenige der Fassung, wenn auch im spannungsfrei eingesetzten Zustand (Fig. 1) ein wenig davon abgehoben, so daß die Kraftübertragung im unverformten Zustand ausschließlich durch die Seitenflächen 5, 6 stattfindet. Der radial vorspringende Rand 23 des Einsatzes hält gleichfalls ein gewisses Spiel gegenüber der Stirnfläche 11 der Fassung ein, um einen spielfreien Sitz der Konusfläche 5 zu ermöglichen.

Zentrisch zur Konus- und Zylinderfläche 5, 6 ist die sphärische Gelenkfläche 14 in dem Einsatz angeordnet. Jedoch soll eine exzentrische Lage nicht ausgeschlossen sein.

Die mit durchgezogenen Linien gezeichnete Stirnfläche 15 des Einsatzes liegt in einer Ebene, die etwas geneigt zu der Ebene der Stirnfläche 11 der Fassung verläuft. Mit anderen Worten verläuft ihre lotrechte Mittelachse 16, die die Richtung der Gelenkfläche 14 angibt, in einem spitzen Winkel 17 zur Fassungsmittelachse 9. Durch Einstellung des Drehwinkels des Einsatzes gegenüber der Fassung läßt sich daher die Richtung der Gelenkfläche weitgehend unterschiedlich einstellen.

Die Basisfläche des Einsatzes 2 enthält am Umfang eine Vielzahl zylindrischer Bohrungen, deren Durchmesser und deren Abstand von der Rotationsachse 9 dem des Zylindervorsprungs 10 der Fassung gleicht. Der Einsatz kann daher in solchen Winkelstellungen in die Fassung eingesetzt werden, in denen jeweils eine dieser Bohrungen den Zylindervorsprung aufnimmt. Der Zylindervorsprung und diese Bohrung bilden dann gemeinsam eine Einrichtung zur Fixierung der relativen Drehstellung von Fassung und Einsatz, solange der Einsatz innerhalb der Fassung gehalten ist.

Letzteres wird durch Verankerungs-Rastzungen 18 am Einsatz gewährleistet, die mit radial vorragenden Nasen hinter den Rand 19 am Boden der Fassung greifen, nachdem sie mit den Schrägflächen der Nasen daran unter elastischer Deformation vorbeigegangen sind.

Die in Fig. 1 und 4 mit durchgezogenen Linien gezeigte Gestalt der Gleitfläche 14 zeigt, daß diese sich von der Stelle ihres größten Durchmessers zu ihrem Öffnungsrand ein wenig verengt. Die Verengung ist so gering, daß der Gelenkkopf unter geringfügiger elastischer Deformation des Einsatzes in die Gelenkfläche eingefügt werden kann. Stattdessen ist es auch möglich, an den Bereich größten Durchmessers der Gelenkfläche öffnungsseitig eine Zylinderfläche 21 anzuschließen, durch die der Gelenkkopf ohne Deformation des Einsatzes eingeführt werden kann. Zweckmäßigerweise wird die Richtung dieser Zylinderfläche, die einen Teil der Gelenkfläche bildet, deren Richtung 16 angepaßt oder sogar noch stärker geneigt, so daß der Teil 22 der Gelenkfläche, der in dem am weitesten vorragenden Bereich 23 des Einsatzes liegt, und der bei der Luxationssicherung eine bedeutende Rolle spielt, bis zur Öffnungskante der Gleitfläche hin ausschließlich sphärisch sein kann.

Die Fassung 1 kann mit einem geeigneten Instrument in das vorbereitete Acetabulum eingeschraubt werden. Dabei kann die günstigste Stellung für eine optimale Verankerung gewählt werden, wobei die Frage, ob diese Stellung auch günstigste Verhältnisse im Hinblick auf die Luxationssicherung und den Bewegungsumfang des Gelenks ergibt, zunächst im Hintergrund bleiben kann. Nachdem die Fassung 1 fest implantiert ist, wird der Kunststoffeinsatz 2 in die Fassung eingesetzt. Dabei kann die Rotationsstellung des Einsatzes unter Beachtung der Lage der Bohrungen 20 frei gewählt werden im Hinblick auf eine solche Lage der Stirnfläche des Einsatzes, die eine optimale Luxationssicherung und den gewünschten Bewegungsumfang des Femurs erwarten läßt.

Es können unterschiedliche Einsätze bereitgehalten werden, die je nach Lage des Falls eine unterschiedlich starke Korrektur der Stellung des Einsatzes gegenüber derjenigen der Fassung erlauben, also beispielsweise Einsätze mit unterschiedlicher Neigung der Stirnfläche 15 oder mit unterschiedlicher Höhe ihres vorragenden Teils.

Die Einrichtungen zur gegenseitigen Rotationssicherung von Fassung und Einsatz können selbstverständlich in anderer Form und Lage als die im Ausführungsbeispiel erläuterten ausgeführt werden. Z. B. kann eine Verzahnung in der Konusverbindung vorgesehen sein.

**Patentansprüche** (für die Vertragsstaaten : AT, BE, LU, NL, SE)

1. Hüftpfannen-Endoprothese bestehend aus einer implantierbaren Fassung (1) und einem darin gehaltenen, die Gelenkfläche (14) bildenden Einsatz (2), wobei die Fassung (1) und der Einsatz (2) zusammenwirkende, rotations- oder sternsymmetrische, auf eine Fassungsmittelachse (9) bezogene Paßflächen (5, 6) aufweisen und die Gelenkfläche (14) durch unterschiedliche Winkeleinstellung der Paßflächen (5, 6) zueinander verstellbar ist, dadurch gekennzeichnet, daß die zur Öffnungsbegrenzung (15) der Gelenkfläche (14) lotrechte Mittelachse (16) von der Richtung der Fassungsmittelachse (9) abweicht, derart, daß die Öffnungsbegrenzung (15) auf einer Seite (23) stärker von der Fassung vorragt, als auf der anderen Seite.

2. Hüftpfannen-Endoprothese nach Anspruch 1, dadurch gekennzeichnet, daß die zur Öffnungsbegrenzung (15) der Gelenkfläche (14) lotrechte Mittelachse (16) einen spitzen Winkel (17) mit der Fassungsmittelachse (9) einschließt.

3. Hüftpfannen-Endoprothese nach Anspruch 1 oder 2, dadurch gekennzeichnet, daß die Paßflächen (5, 6) zwischen Fassung (1) und Einsatz (2) von der sphärischen Form abweichende Rotationsflächen um die Fassungsmittelachse (9) sind und daß die Fassung (1) und der Einsatz (2) mit ineinandergreifenden Erhöhungen (10) und Vertiefungen (20) am äußeren Rand einer an der größten Eindringtiefe der Paßflächen (5, 6) in die Fassung (1) angeordneten Basisflächen (7, 12) versehen sind.

**Patentanspruch** (für die Vertragsstaaten : CH, DE, FR, GB, LI)

Hüftpfannen-Endoprothese mit folgenden Merkmalen :

a) sie umfaßt eine implantierbare Fassung (1) mit einer Mittelachse (9) und einen in der Fassung gehaltenen, die Gelenkfläche (14) bildenden Einsatz (2),

b) die zur Öffnungsbegrenzung (15) der Gelenkfläche (14) lotrechte Mittelachse (16) weicht von der Richtung der Fassungsmittelachse (9) ab, derart, daß die Öffnungsbegrenzung (15) auf einer Seite (23) stärker von der Fassung (1) vorragt als auf der anderen Seite,

c) die Fassung (1) und der Einsatz (2) weisen zusammenwirkende, rotations- oder sternsymmetrische auf die Fassungsmittelachse (9) bezogene Paßflächen (5, 6) auf,

d) die Gelenkfläche (14) ist durch unterschiedliche Winkeleinstellung der Paßflächen (5, 6) zueinander verstellbar,

e) die Paßflächen (5, 6) sind von der sphärischen Form abweichende Rotationsflächen um die Fassungsmittelachse (9) und

f) die Fassung (1) und der Einsatz (2) sind mit ineinandergreifenden Erhöhungen (10) und Vertiefungen (20) versehen, die am äußeren Rand einer in der Tiefe der Fassung liegenden Basisfläche (7, 12) angeordnet sind.

**Claims** (for the Contracting States : AT, BE, LU, NL, SE)

1. An endoprosthesis for the cotyloid cavity, comprising an implantable socket member (1) and an insert (2) retained therein forming the articulation surface (14), wherein the socket member (1) and the insert (2) have cooperating mating surfaces (5, 6) which are rotationally symmetrical or radially symmetrical relative to a medial axis (9) of the socket member, and the articulation surface (14) is displaceable by varying the angular setting of the mating surfaces (5, 6) relative to one another, characterised in that the medial axis (16) perpendicular to the opening boundary (15) of the articulation surface (14) deviates from the direction of the medial axis (9) of the socket member in such a way that the opening boundary (15) protrudes to a greater extent from the socket member (1) on one side (23) than on the other side.

2. An endoprosthesis for the cotyloid cavity according to Claim 1, characterised in that the medial axis (16) perpendicular to the opening boundary (15) of the articulation surface (14) forms an acute angle (17) with the medial axis (9) of the socket member.

3. An endoprosthesis for the cotyloid cavity according to Claim 1 or 2, characterised in that the mating surfaces (5, 6) between the socket member (1) and the insert (2) are surfaces of revolution about the medial axis (9) of the socket member deviating from a spherical shape, and the socket member (1) and the insert (2) are provided with inter-engaging projections (10) and recesses (20) on the outer edge of a base surface (7, 12) arranged at the maximum penetration depth of the mating surfaces (5, 6) into the socket member (1).

**Claim** (for the Contracting States : CH, DE, FR, GB, LI)

An endoprosthesis for the cotyloid cavity, having the following features :

a) it comprises an implantable socket member (1) with a medial axis (9), and an insert (2) retained in the socket member and forming the articulation surface (14),

b) the medial axis (16) perpendicular to the opening boundary (15) of the articulation surface (14) deviates from the direction of the medial axis (9) of the socket member in such a way that the opening boundary (15) protrudes to a greater extent from the socket member (1) on one side (23) than on the other side,

c) the socket member (1) and the insert (2) have cooperating mating surfaces (5, 6) which are rotationally symmetrical or radially symmetrical relative to the medial axis (9) of the socket member,

d) the articulation surface (14) is displaceable by varying the angular setting of the mating surfaces (5, 6) relative to one another,

e) the mating surfaces (5, 6) are surfaces of revolution about the medial axis (9) of the socket member deviating from a spherical shape, and

f) the socket member (1) and the insert (2) are provided with inter-engaging projections (10) and recesses (20) which are disposed on the outer edge of a base surface (7, 12) situated at the bottom of the socket member.

**Revendications** (pour les Etats contractants : AT, BE, LU, NL, SE)

1. Endoprothèse de cavité cotyloïde, se composant d'une monture implantable (1) et d'une garniture (2) maintenue dans celle-ci et formant la surface articulaire (14), la monture (1) et la garniture (2) comportant des surfaces (5, 6) d'adaptation par rapport à un axe (9) de la monture, présentant une symétrie de révolution ou stellaire et coopérant entre elles, et la surface articulaire (14) étant réglable par positionnement angulaire différent des surfaces d'adaptation (5, 6) l'une par rapport à l'autre, caractérisée en ce que la ligne centrale (16) perpendiculaire à la limite de l'ouverture (15) de la surface articulaire (14) s'écarte de la direction de l'axe (9) de la monture, de telle manière que d'un côté, la limite de l'ouverture (15) fasse plus fortement saillie sur la monture que de l'autre côté.

2. Endoprothèse de cavité cotyloïde selon la revendication 1, caractérisée en ce que la ligne centrale (16) perpendiculaire à la limite de l'ouverture (15) de la surface articulaire (14) forme un angle aigu (17) avec l'axe (9) de la monture.

3. Endoprothèse de cavité cotyloïde selon la revendication 1 ou 2, caractérisée en ce que les surfaces d'adaptation (5, 6) entre la monture (1) et la garniture (2) sont des surfaces de révolution autour de l'axe (9) de la monture s'écartant de la forme sphérique, et en ce que la monture (1) et la garniture (2) sont munies de reliefs (10) et de creux (20) qui s'engagent les uns dans les autres, au niveau du bord extérieur d'une surface de base (7, 12) située à la plus grande profondeur de pénétration des surfaces d'adaptation (5, 6) dans la monture (1).

**Revendication** (pour les Etats contractants : CH, DE, FR, GB, LI)

Endoprothèse de cavité cotyloïde, présentant les caractéristiques suivantes :

a) elle comprend une monture implantable (1) qui présente un axe (9), ainsi qu'une garniture (2) qui est maintenue dans la monture et forme la surface articulaire (14),

b) la ligne centrale (16) perpendiculaire à la limite de l'ouverture (15) de la surface articulaire (14) s'écarte de la direction de l'axe (9) de la monture, de telle manière que d'un côté (23), la limite de l'ouverture (15) fasse plus fortement saillie sur la monture (1) que de l'autre côté,

c) la monture (1) et la garniture (2) comportent des surfaces (5, 6) d'adaptation par rapport à l'axe (9) de la monture, présentant une symétrie de révolution ou stellaire et coopérant entre elles,

d) la surface articulaire (14) est réglable par positionnement angulaire différent des surfaces d'adaptation (5, 6) l'une par rapport à l'autre,

e) les surfaces d'adaptation (5, 6) sont des surfaces de révolution autour de l'axe (9) de la monture, s'écartant de la forme sphérique et

f) la monture (1) et la garniture (2) sont munies de reliefs (10) et de creux (20) qui s'engagent les uns dans les autres et qui sont disposés au niveau du bord extérieur d'une surface de base (7, 12) située au fond de la monture.

Fig.1

Fig.4

Fig.2

Fig.5

Fig.3